# EUROPÄISCHE PATENTSCHRIFT

(11) **EP 3 549 562 B1**
(45) Veröffentlichungstag und Bekanntmachung des Hinweises auf die Patenterteilung: **29.07.2020**
(21) Anmeldenummer: 18020129.5
(22) Anmeldetag: 03.04.2018
(51) Int. Cl.: A61F 13/02

(54) **HAFTBAND FÜR KLEIDUNGSSTÜCKE, INSBESONDERE FÜR MEDIZINISCHE KOMPRESSIONSSTRÜMPFE ODER BANDAGEN**
ADHESIVE STRIP FOR GARMENTS, IN PARTICULAR FOR MEDICAL COMPRESSION STOCKINGS OR BANDAGES
BANDE ADHÉSIVE POUR VÊTEMENTS, EN PARTICULIER POUR BAS DE CONTENTION MÉDICAL OU POUR BANDAGES

(43) Veröffentlichungstag der Anmeldung: 09.10.2019
(73) Patentinhaber: medi GmbH & Co. KG, 95448 Bayreuth (DE)
(72) Erfinder: Hoffeins, Peter, 95448 Bayreuth (DE)

(56) Entgegenhaltungen:
- DE-C2- 2 505 923
- DE-U1-202004 014 731

## Beschreibung

Die vorliegende Erfindung bezieht sich auf ein Haftband für Kleidungsstücke, insbesondere für medizinische Kompressionsstrümpfe oder Bandagen nach dem Oberbegriff des Anspruchs 1 sowie auf ein Kleidungsstück, insbesondere auf einen medizinischen Kompressionsstrumpf oder Bandage ach Anspruch 11.

Derartige Kleidungsstücke, insbesondere Beinbekleidungsstücke, wie zum Beispiel Kompressionsstrümpfe, dienen insbesondere dazu gezielt Druck auf den Körper eines Patienten auszuüben. Ziel ist es ein geschädigte Venen- und/ oder Lymphsystem eines Patienten zu entlasten. Bandagen werden zur Stabilisierung, Entlastung, Ruhigstellung, Führung und/ oder Korrektur von Gliedmaßen eines Patienten verwendet. Zur Ausbildung dieser medizinischen Kleidungsstücke werden bevorzugt sogenannte kompressive Gestricke verwendet, die mittels einer Flach- oder Rundstrickmaschine flach- oder rundgestrickt werden.

Um das Verrutschen der, insbesondere kompressiven, Kleidungsstücke an einem Patienten zu vermeiden, sind sogenannte Haftbänder bzw. Spitzenhaftbänder bekannt. Diese werden randseitig an den Kleidungsstücken, insbesondere an deren kompressiven Gestricken, befestigt, insbesondere angenäht. Die Haftbänder selbst sind üblicherweise aus einem in Längsrichtung elastischen und in Querrichtung eher unelastischen Material gefertigt. Durch die Längselastizität der Haftbänder, stehen diese einem Anziehen der Kleidungsstücke nicht im Wege. Die geringe Querelastizität und eine Haftwirkung gewährleisten einen sichereren Halt des, insbesondere kompressiven, Kleidungsstücks am Körper des Patienten.

Eine derartiges Haftband für ein Unterbekleidungsstück, insbesondere für orthopädische Zwecke, ist aus der DE 20 2004 014 731 U1 bekannt.

Das bekannte Spitzenhaftband ist aus einem längselastischen Grundgewirke mit einem konstanten Querschnitt gebildet und bevorzugt über eine Nahtverbindung mit einem Kleidungsstück verbunden, in dem es randseitig über das Kleidungsstück ragt, also überlappend angeordnet und vernäht ist. Die auf der Haut zu tragende Seite des Spitzenhaftbandes weist eine Haftschicht auf, die aus zwei längs aufgebrachten Silikonstreifen besteht. Durch die an der Haut anliegende Haftschicht wird ein Verrutschen des Kleidungsstückes vermieden.

Auch die DE 25 05 923 C2 offenbart ein Haftband für Kleidungsstücke.

Das elastische Haftband weist einen elastischen Grundkörper aus einem textilen Gewebe auf, an dem, insbesondere an dessen Innenseite, zusätzlich ein nicht gleitendes Elastomer aufgebracht ist. Das Band selbst ist über eine Nahtverbindung mit dem Kleidungsstück verbunden. Hierzu kann das Kleidungsstück wahlweise an der Innenseite des Bandes angenäht sein oder stirnseitig an dem Band anliegen und über eine Naht mit dem elastischen Band verbunden sein. Auch dieses Band weist einen elastischen Grundkörper mit einen über seine gesamte Breite konstanten Querschnitt auf.

Als nachteilig dieser Ausgestaltung der Haftbänder bzw. Spitzhaftbänder erweist sich die Verbindung zwischen Haftband und Kleidungsstück. Die bekannte Ausbildung der elastischen Grundkörper der Haftbänder mit einem konstanten Querschnitt über die gesamte Breite der Bänder sowie die Befestigung der Kleidungsstücke an der Innenseite des Grundkörpers der Haftbänder, führt zu einer auftragenden Verbindungsstelle. Das Kleidungsstück sowie das Nahtmaterial steht über die Innenseite des Haftbandes hinaus. Dies führt zu einer unerwünschten Wulstbildung, welche beim Tragen der Kleidungsstücke für den Patienten störend empfunden wird.

Auch die aus dem Stand der Technik, insbesondere aus der DE 25 05 923 C2, bekannte stirnseitige bzw. Stoß-an-Stoß Verbindung des Kleidungsstücks mit dem Haftband, insbesondere mit dessen Grundkörper, um eine auftragende Verbindung möglichst zu vermeiden, bringt Nachteile mit sich. Durch das Ziehen an dem Haftband, insbesondere beim Anziehen von kompressiven Kleidungsstücken mit hohen Zugkräften, entstehen sichtbare Lücken zwischen Haftband und Kleidungsstück. Diese Lücken in dem Kleidungsstück sind unerwünscht. Des Weiteren weist eine derartige Nahtverbindung im Vergleich zur überlappenden Befestigung des Kleidungsstücks an dem Haftband eine geringere Nahtfestigkeit auf.

Der vorliegenden Erfindung liegt die Aufgabe zugrunde ein Haftband für Kleidungsstücke, insbesondere für medizinische Kompressionsstrümpfe oder Bandagen, zu schaffen, welches die Nachteile aus dem Stand der Technik vermeidet, insbesondere den Tragekomfort des Haftbandes, die Optik im Nahtbereich, insbesondere im getragenen Zustand, sowie die Nahthaltbarkeit und Nahtfestigkeit verbessert.

Gemäß einem Ausführungsbeispiel des Haftbandes für Kleidungsstücke, insbesondere für medizinische Kompressionsstrümpfe oder Bandagen, weist dieses einen flexiblen Grundkörper mit einer dem Kleidungsstück zugewandten Innenseite sowie einer Außenseite auf, wobei der flexible Grundkörper an der Innenseite neben einem Haftabschnitt einen Befestigungsabschnitt für das Kleidungsstück aufweist, und wobei der Befestigungsabschnitt durch eine randseitige Ausnehmung in dem flexiblen Grundkörper ausgebildet ist, so dass das Kleidungsstück an dem Haftband, insbesondere höhenmäßig, versetzt zu dem Haftabschnitt des flexiblen Grundkörpers in der Ausnehmung befestigbar ist.

Gemäß einem ersten Ausführungsbeispiel ist der flexible Grundkörper aus einem textilen Gewebe, bestehend aus mehreren Kettfäden und zumindest einem zwischen den Fäden eingearbeiteten Schussfaden, gebildet. Alternativ ist es auch möglich, dass das Haftband aus einem gewirkten oder geraschalten Grundkörper gebildet ist. Handelt es sich um ein Gewebe, dann erstreckt sich der Schussfaden des textilen Gewebes bevorzugt im Wesentlichen über die gesamte Breite des Haftbandes. D.h. der Schussfaden ist in dem flexiblen Grundkörper sowohl in dem Haftabschnitt als auch in dem Befestigungsabschnitt angeordnet. Dies hat den Vorteil, dass der Befestigungsabschnitt fest mit dem Haftabschnitt verbunden ist und dadurch die Nahthaltbarkeit deutlich verbessert wird, was insbesondere beim Anziehen von kompressiven Kleidungsstücken mit hohen Zugkräften von besonders großer Bedeutung ist.

Bevorzugt ist der flexible Grundkörper des Haftbandes in Längsrichtung elastisch und in Querrichtung im Wesentlichen unelastisch, so dass das Haftband ein Anziehen eines daran befestigten Kleidungsstücks nicht erschwert und gleichzeitig einen sicheren Halt des, insbesondere kompressiven, Kleidungsstücks am Körper des Patienten ermöglicht.

Gemäß einem zweiten Ausführungsbeispiel des Haftbandes ist auf dem Haftabschnitt des flexiblen Grundkörpers zumindest bereichsweise eine rutschhemmende Beschichtung aufgebracht. Diese ist bevorzugt durch musterförmige, insbesondere noppenförmige oder linienförmige, Haftbereiche ausgebildet. Die Beschichtung weist hierbei bevorzugt eine Höhe von ungefähr 0,5 mm auf. Insbesondere die versetzte Anordnung des Befestigungsabschnitts zu dem Haftabschnitt, insbesondere eines an dem Haftband befestigbaren Kleidungsstücks in der randseitigen Ausnehmung in dem flexiblen Grundkörper, ermöglicht eine besonders flache Ausbildung der rutschhemmenden Beschichtung, da ein Hervorstehen der Beschichtung gegenüber der Innenseite eines an dem Haftband befestigen Kleidungsstücks leicht möglich ist. Dies bringt den Vorteil mit sich, dass Druckstellen in der Haut, auf Grund stark hervortretender Muster, insbesondere Noppen, durch die rutschhemmende Beschichtung weitestgehend vermieden werden.

Zur Befestigung eines Kleidungsstücks, insbesondere eines Gestricks, in der Ausnehmung des flexiblen Grundkörpers des Haftbandes weist die Ausnehmung bevorzugt eine Breite von mindestens ungefähr 5 mm auf. Die Höhe der Ausnehmung beträgt bevorzugt ungefähr 0,5 bis 1,5 mm.

Gemäß einem weiteren Ausführungsbeispiel des Haftbandes ist der flexible Grundkörper einstückig ausgebildet. Hierbei sind der Haftabschnitt und der Befestigungsabschnitt aus einem einzigen Grundkörper gebildet. Alternativ ist es aber auch möglich, dass der Befestigungsabschnitt, insbesondere in Form einer Lippe, an dem Haftabschnitt des Grundkörpers befestigt ist. Möglich wäre hierbei, dass die Lippe an dem Haftabschnitt angenäht oder aufgeschweißt ist. Gemäß der bevorzugten Variante, nämlich der einstückigen Ausbildung des flexiblen Grundkörpers, weist der flexible Grundkörper des Weiteren bevorzugt an seiner Außenseite über seine gesamte Breite eine durchgängige Oberfläche auf. D.h. es befindet sich an der Außenseite bevorzugt kein Versatz, Stufe oder Rücksprung zwischen Haftabschnitt und Befestigungsabschnitt. Zusätzlich können natürlich an der Außenseite des flexiblen Grundkörpers diverse Materialen aufgebracht oder in die Oberfläche des Grundkörpers eingebracht bzw. eingearbeitet sein, um gemusterte Oberflächen zu erzeugen.

Gemäß einem Ausführungsbeispiel eines Kleidungsstücks, insbesondere medizinischen Kompressionsstrumpfes oder Bandage, weist dieses ein Haftband mit einer randseitigen Ausnehmung gemäß einem der vorherigen Ausführungsbeispiele auf.

Hierzu ist das Kleidungsstück bevorzugt durch eine formschlüssige Verbindung, insbesondere mittels einer Nahtverbindung, an dem Haftband befestigt. Alternativ wäre auch eine stoffschlüssige Verbindung, insbesondere Schweißen, Kleben und/ oder Vulkanisieren möglich. Die Mindestanforderung an die vorgeschlagenen oder an weitere alternative Befestigungsmethoden ist, dass diese die Zugkräfte, die durch das Ziehen an dem Haftband beim Anziehen des Kleidungsstücks entstehen, standhält.

Des Weiteren ist bevorzugt nur in dem randseitigen Befestigungsabschnitt des Haftbandes eine Verbindung mit dem Kleidungsstück ausgeführt. Die Verbindung erstreckt sich also hierbei nicht in den Haftabschnitt, sondern ist lediglich in dem dazu höhenmäßig zurückversetzten Befestigungsabschnitt ausgebildet. Die Breite der Naht kann hierbei aber variieren und sich über den Befestigungsabschnitt hinweg in das Kleidungsstück hinein erstrecken.

Gemäß einem weiteren Ausführungsbeispiel des Kleidungsstücks, ist die Verbindung des Kleidungsstücks mit dem Haftband derart ausgebildet, dass die Innenseite des Kleidungsstücks im Wesentlichen bündig zu dem Haftabschnitt des flexiblen Grundkörpers des Haftbandes angeordnet ist. Durch die Ausbildung der Ausnehmung in dem Haftband entsprechend der Stärke bzw. Dicke des Kleidungsstücks, insbesondere einer Gestrickstärke, wird ein Überstand des Kleidungsstücks über die Innenseite des Haftabschnitts des Haftbandes hinaus vermieden. Druckstellen, auf Grund der Befestigung des Kleidungsstücks an dem Haftband, treten somit beim Tragen des Kleidungsstücks nicht auf.

Gemäß einem weiteren Ausführungsbeispiel des Kleidungsstücks, ist die Verbindung des Kleidungsstücks mit dem Haftband derart ausgebildet ist, dass die auf dem flexiblen Grundkörper des Haftbandes aufgebrachte rutschhemmende Beschichtung über die Innenseite des Kleidungsstücks hinaussteht. Die Beschichtung weist hierbei bevorzugt eine Höhe von ungefähr 0,5 mm auf. Wie bereits zuvor erwähnt, ermöglicht die versetzte Anordnung des Kleidungsstücks in der randseitigen Ausnehmung in dem flexiblen Grundkörper, eine besonders flache Ausbildung der rutschhemmenden Beschichtung, da ein Hervorstehen der Beschichtung gegenüber der Innenseite des Kleidungsstücks leicht möglich ist.

Das vorliegende Haftband für Kleidungsstücke, insbesondere für medizinische Kompressionsstrümpfe oder Bandagen, zeichnet sich durch eine Reihe erheblicher Vorteile aus.

Durch die Ausbildung des Haftbandes durch einen flexiblen Grundkörper mit einem Haftabschnitt und einem Befestigungsabschnitt, wobei der Befestigungsabschnitt durch eine randseitige Ausnehmung in dem flexiblen Grundkörper ausgebildet ist, so dass ein Kleidungsstück an dem Haftband versetzt zu dem Haftabschnitt in der Ausnehmung befestigbar ist, wird der Tragekomfort des Haftbandes, insbesondere des damit verbundenen Kleidungsstücks, wesentlich erhöht. Eine unerwünschte Wulstbildung, wie sie aus dem Stand der Technik bekannt ist und welche beim Tragen der Kleidungsstücke für den Patienten als störend empfunden wird, wird durch das erfindungsgemäße Haftband vermieden.

Einen weiteren Vorteil der Erfindung bildet, gegenüber der stirnseitigen bzw. Stoß-an-Stoß Verbindung des Kleidungsstücks mit dem Haftband, bei der ein überlappende Befestigung möglichst vermieden wird, um Druckstellen zu vermeiden, die deutlich verbesserte Nahthaltbarkeit bzw. Nahtfestigkeit, was insbesondere beim Anziehen von kompressiven Kleidungsstücken mit hohen Zugkräften von besonders großer Bedeutung ist. Bei dem erfindungsgemäßen Haftband werden die Vorteile einer überlappenden Verbindung mit der einer stirnseitigen bzw. Stoß-an-Stoß Verbindung vereint.

Ein weiterer Vorteil der sich aus dem erfindungsgemäßen Haftband ergibt ist, dass eine deutlich bessere Optik im Befestigungsbereich, insbesondere im Nahtbereich bei Verwendung einer Nahtverbindung, gewährleistet wird. Bei der aus dem Stand der Technik bekannten stirnseitigen bzw. Stoß-an-Stoß Verbindung kommt es auf Grund der großen Zugkräfte beim Anziehen des mit dem Haftband verbundenen Kleidungsstücks zu einem Aufdehnen der Naht, was einen sichtbaren Spalt bzw. Lücke zwischen Haftband und Kleidungsstück zur Folge hat. Durch das erfindungsgemäße Haftband, insbesondere durch die Möglichkeit der integrierten Befestigung des Kleidungsstücks an bzw. in dem Haftband, wird eine derartige Spaltbildung, insbesondere beim Anziehen der Kleidungsstücke, verhindert.

Nachfolgend wird die Erfindung anhand mehrere Ausführungsbeispiele und in Verbindung mit den beigefügten Zeichnungen erläutert.

Dabei zeigt:
Figur 1 ein erstes Ausführungsbeispiel des Haftbandes für Kleidungsstücke, insbesondere für medizinische Kompressionsstrümpfe oder Bandagen, in der Draufsicht,
Figur 2 eine Schnittdarstellung des in Figur 1 gezeigten ersten Ausführungsbeispiels des Haftbandes für Kleidungsstücke,
Figur 3 eine dreidimensionale Darstellung eines zweiten Ausführungsbeispiels des Haftbandes, angebracht an einem Kleidungsstück, wobei das Haftband bereichsweise aufgeschnitten ist,
Figur 4 eine Schnittdarstellung des in Figur 3 gezeigten zweiten Ausführungsbeispiels des Haftbandes für Kleidungsstücke;

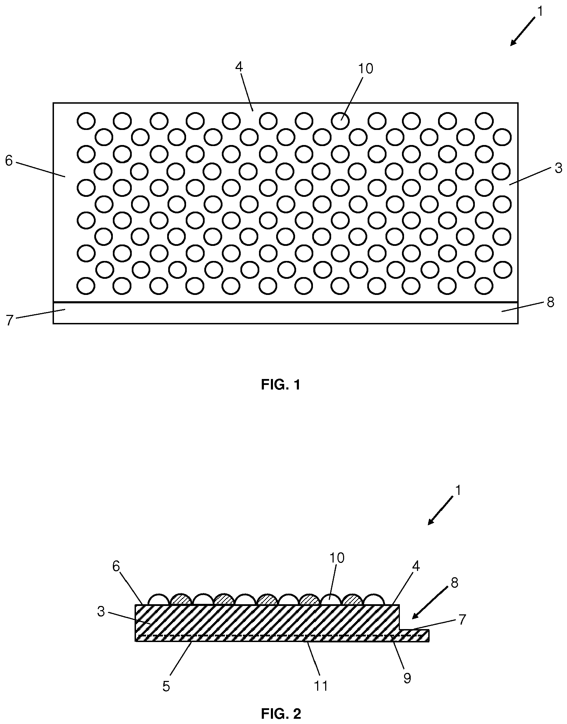

Das in Figur 1 schematisch dargestellte Haftband 1 für Kleidungsstücke, insbesondere für medizinische Kompressionsstrümpfe oder Bandagen, umfasst einen flexiblen Grundkörper 3 mit einer einem Kleidungsstück zugewandten Innenseite 4. Die Innenseite 4 besteht aus einem Haftabschnitt 6 und einem Befestigungsabschnitt 7. Der Befestigungsabschnitt 7 ist durch eine randseitige Ausnehmung 8 in dem flexiblen Grundkörper 3 ausgebildet. Die Ausnehmung 8 umfasst hierbei bevorzugt eine Breite von mindestens ungefähr 5 mm. Die Höhe der Ausnehmung 8 beträgt bevorzugt mindestens ungefähr 0,5 mm. Als randseitig wird ein Abschnitt verstanden, welcher sich am oberen oder unteren Rand des Haftbandes 1 befindet und sich bis an das obere oder untere Ende des Haftbandes 1 erstreckt. Der Befestigungsabschnitt 7 ist also nicht beidseitig von einem Haftabschnitt 6 umgeben und ist somit nicht im Haftbandinneren angeordnet. Durch die randseitige Ausnehmung 8 ist ein Kleidungsstück, insbesondere ein Gestrick, an dem Haftband 1 höhenmäßig versetzt zu dem Haftabschnitt 6 des flexiblen Grundkörpers 3 in der Ausnehmung 8 befestigbar. Der Haftabschnitt 6 umfasst bevorzugt eine rutschhemmende Beschichtung 10, welche auf den Haftabschnitt 6 aufgebracht ist. Diese ist in diesem Ausführungsbeispiel als noppenförmige Haftbereiche ausgebildet.

In Figur 2 ist das Haftband 1 für ein Kleidungsstück aus Figur 1 in einer Schnittdarstellung gezeigt. Der Querschnitt zeigt, dass der Haftabschnitt 6 und der Befestigungsabschnitt 7 durch eine randseitige Ausnehmung bzw. Rücksprung 8 höhenmäßig versetzt zueinander in bzw. an dem flexible Grundkörper 3 ausgebildet sind. Durch die Ausnehmung 8, welche an der der Haut eines Patienten zugewandten Seite 4 des Haftbandes 1 ausgebildet ist, wird an dem Haftband 1, insbesondere an dem flexiblen Grundkörper 3, ein lippenförmiger Befestigungsabschnitt 7 gebildet. Der Befestigungsabschnitt bzw. die Lippe 7 mit einem gegenüber dem übrigen Querschnitt des Haftbandes 1 verringerten Querschnitt ist hierbei bevorzugt einteilig mit dem flexiblen Grundkörper 3 ausgebildet. Alternativ ist es möglich, dass der den verringerten Querschnitt aufweisende Teil des flexiblen Grundkörpers 3 an dem Haftband 1 angeklebt, angenäht oder angewebt ist.

Der flexible Grundkörper 3 ist bevorzugt einteilig aus einem textilen Gewebe, bestehend aus mehreren Kettfäden und zumindest einem zwischen den Fäden eingearbeiteten Schussfaden 9 gebildet. Hierbei erstreckt sich der zumindest eine eingearbeitete Schussfaden 9 im Wesentlichen über die gesamte Breite des Haftbandes 1. Dieser ist also sowohl im Haftabschnitt 6 als auch im Befestigungsabschnitt 7 angeordnet, wodurch der Halt des Befestigungsabschnittes 7 an dem Haftabschnitt 6, also der Lippe an dem Haftband 1, deutlich erhöht wird. Der Grundkörper 3 selbst ist bevorzugt in Längsrichtung elastisch und in Querrichtung im Wesentlichen unelastisch.

Ebenfalls in der Schnittdarstellung ist die rutschhemmende Beschichtung 10 an der Innenseite 4 des Haftbandes 1, insbesondere ein Schnitt durch die noppenförmigen Haftbereiche, gezeigt. An der gegenüberliegenden Außenseite 5 des Haftbandes 1 weist dieses eine, insbesondere einzige, durchgängige Oberfläche 11 auf. Diese Fläche 11, welche bevorzugt im Wesentlichen ohne Vor- oder Rücksprünge ausgebildet ist, bildet eine gemeinsame Außenseite 5 für den Haftabschnitt 6 und den Befestigungsabschnitt 7. Dies hat den Vorteil, dass ein Kleidungsstück, insbesondere ein Gestrick, sehr unauffällig und in einer nicht auftragenden Art und Weise an dem Haftband 1 befestigbar ist.

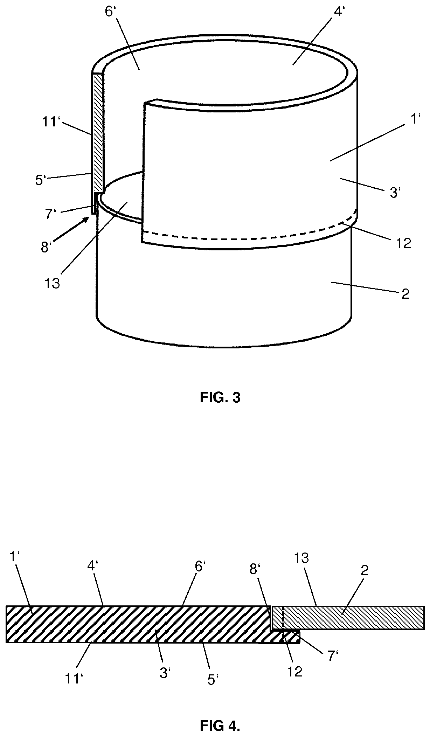

In Figur 3 ist ein zweites Ausführungsbeispiel des Haftbandes 1', angebracht an einem Kleidungsstück 2, wobei das Haftband 1' bereichsweise aufgeschnitten ist, in einer dreidimensionalen Darstellung gezeigt. Das Kleidungsstück 2 ist bevorzugt ein medizinischer rund oder flachgestrickter Arm- oder Beinstrumpf, insbesondere Kompressionsstrumpf, bestehend aus einem kompressiven Strickteil, um ein geschädigtes Venen- und/ oder Lymphsystem eines Patienten zu entlasten. Derartige Kompressionsstrümpfe für das Bein bestehen aus einem Fußteil sowie Wadenteil. Alternativ ist es auch denkbar, dass das Fußteil und das Wadenteil lösbar miteinander verbunden sind. Der Kompressionsstrumpf ist bevorzugt einer der normierten vier Kompressionsklassen zugeordnet. Das Kleidungsstück 2, insbesondere der halterlose Kompressionsstrumpf, ist bevorzugt mittels einer Nahtverbindung 12 mit dem Haftband 1' formschlüssig verbunden, um einen sicheren Halt des Strumpfes an einem Bein oder Arm eines Patienten zu gewährleisten.

Auch in diesem Ausführungsbeispiel weist das Haftband 1' einem flexiblen Grundkörper 3' mit einer Innenseite 4' und einer Außenseite 5' auf. An der Außenseite 5' können zu dekorativen Zwecken diverse Muster in den Grundkörper 3' eingearbeitet oder an diesem aufgebracht sein. An der Innenseite 4' weist das Haftband einen Haftabschnitt 6' sowie einen Befestigungsabschnitt 7' auf. Der Haftabschnitt 6' ist hierbei bevorzugt ohne eine zusätzliche rutschhemmende Beschichtung ausgebildet. In diesem Ausführungsbeispiel weist das Material des flexiblen Grundkörpers 3' bevorzugt selbst rutschhemmende Eigenschaften auf. Der Befestigungsabschnitt 7' ist durch die randseitige Ausnehmung 8' gebildet, welche sich umlaufend um das gesamte Haftband 1', insbesondere um den gesamten Haftbandabschnitt 1' erstreckt, welcher benötigt wird, um einen umlaufenden Haftbereich an dem hülsenförmigen Kleidungsstück 2 auszubilden. Das längliche Haftband 1' wird bevorzugt mittels einer Quernaht geschlossen, um einen hülsenförmigen Bereich an dem Kleidungsstück 2 auszubilden. Die Ausnehmung 8' ist derart ausgebildet, dass das Kleidungsstück 2 daran angenäht werden kann und dieses zur Gänze in der Ausnehmung 8' aufnehmbar ist, so dass es im Wesentlichen nicht über die Innenseite 4' des Haftbandes 1' hinausragt. Die Innenseite 13 des Kleidungsstücks 2 ist somit im Wesentlichen flächenbündig mit der Innenseite 4' an dem Haftband 1' befestigt. An der Außenseite 5' weist das Haftband 1' auch in diesem Ausführungsbeispiel eine durchgängige Oberfläche 11' auf.

In Figur 4 ist das zweite Ausführungsbeispiel des Haftbandess 1', welches mit einem Kleidungsstück 2 verbunden ist, in einer Schnittdarstellung gezeigt. Wie zuvor beschrieben, ist das Kleidungsstück 2 an dem Haftband 1' mittels einer Nahtverbindung 12 derart angebracht, dass die Innenseite 13 des Kleidungsstücks 2 im Wesentlichen flächenbündig zu der Innenseite 4' des flexiblen Grundkörpers 3' des Haftbandes 1' ist, d.h. nur mit einem geringen möglichen Überstand oder rückwertigen Versatz an dem Haftband 1' befestigt ist. Der Befestigungsabschnitt 7' ist in diesem Ausführungsbeispiel ebenfalls durch eine randseitige Ausnehmung 8' in Form eines Rücksprungs bzw. Stufe in dem flexiblen Grundkörper 3', ausgebildet. Die stufenförmige Ausbildung des Grundkörpers 3' ermöglicht den höhemäßigen Versatz zwischen Haftabschnitt 6' und Befestigungsabschnitt 7'. Weitere Formen der Ausnehmung 8' sind selbstverständlich möglich. Bevorzugt wird jedoch eine Ausnehmung in Form einer Stufe. Der Haftabschnitt 6' ist in diesem Ausführungsbeispiel durch einen rutschhemmenden Grundkörper 3' ausgebildet, d.h. dass das Material des flexiblen Grundkörpers 3' selbst bereits rutschhemmende Eigenschaften aufweist. Eine zusätzliche rutschhemmende Beschichtung ist daher nicht notwendig. Ist der Grundkörper 3' aus einem textilen Gewebe gefertigt, so weisen bevorzugt bereits die mehreren Kettfäden des Gewebes rutschhemmende Oberflächen auf. Auch hier gut zu sehen ist, dass das Haftband 1' im Bereich des Haftabschnitts 6' und des Befestigungsabschnitts 7' an der Außenseite 5' eine durchgängige Oberfläche 11' aufweist, so dass das Kleidungsstück 2 in einer nicht auftragenden Art und Weise an dem Haftband 1' befestigbar ist.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Haftband (1, 1') für Kleidungsstücke (2), insbesondere für medizinische Kompressionsstrümpfe oder Bandagen, aufweisend, einen flexiblen Grundkörper (3, 3') mit einer dem Kleidungsstück (2) zugewandten Innenseite (4, 4') sowie einer Außenseite (5, 5'), wobei der flexible Grundkörper (3, 3') an der Innenseite (4, 4') neben einem Haftabschnitt (6, 6') einen Befestigungsabschnitt (7, 7') für das Kleidungsstück (2) aufweist, **dadurch gekennzeichnet, dass** der Befestigungsabschnitt (7, 7') durch eine randseitige Ausnehmung (8, 8') in dem flexiblen Grundkörper (3, 3') ausgebildet ist, so dass das Kleidungsstück (2) an dem Haftband (1, 1') versetzt zu dem Haftabschnitt (6, 6') des flexiblen Grundkörpers (3, 3') in der Ausnehmung (8, 8') befestigbar ist.

2. Haftband (1, 1') für Kleidungsstücke (2) nach Anspruch 1, **dadurch kennzeichnet, dass** der flexible Grundkörper (3, 3') aus einem textilen Gewebe, bestehend aus mehreren Kettfäden und zumindest einem zwischen den Fäden eingearbeiteten Schussfaden (9), gebildet ist.

3. Haftband (1) für Kleidungsstücke (2) nach Anspruch 2, **dadurch kennzeichnet, dass** sich der Schussfaden (9) des textilen Gewebes über die gesamte Breite des Haftbandes (1, 1') erstreckt.

4. Haftband (1, 1') für Kleidungsstücke (2) nach einem der vorherigen Ansprüche, **dadurch kennzeichnet, dass** der flexible Grundkörper (3, 3') in Längsrichtung elastisch und in Querrichtung unelastisch ist.

5. Haftband (1) für Kleidungsstücke (2) nach einem der vorherigen Ansprüche, **dadurch kennzeichnet, dass** auf dem Haftabschnitt (6) des flexiblen Grundkörpers zumindest bereichsweise eine rutschhemmende Beschichtung (10) aufgebracht ist.

6. Haftband (1) für Kleidungsstücke (2) nach Anspruch 5, **dadurch kennzeichnet, dass** die rutschhemmende Beschichtung (10) durch musterförmige, insbesondere noppenförmige oder linienförmige, Haftbereiche ausgebildet ist.

7. Haftband (1, 1') für Kleidungsstücke (2) nach einem der vorherigen Ansprüche, **dadurch kennzeichnet, dass** die Breite der Ausnehmung (8, 8') mindestens 5 mm beträgt.

8. Haftband (1, 1') für Kleidungsstücke (2) nach einem der vorherigen Ansprüche, **dadurch kennzeichnet, dass** die Höhe der Ausnehmung (8,8') mindestens 0,5 mm beträgt.

9. Haftband (1, 1') für Kleidungsstücke (2) nach einem der vorherigen Ansprüche, **dadurch kennzeichnet, dass** der flexible Grundkörper (3, 3') einstückig ausgebildet ist.

10. Haftband (1, 1') für Kleidungsstücke (2) nach einem der vorherigen Ansprüche, **dadurch kennzeichnet, dass** der flexible Grundkörper (3, 3') an seiner Außenseite (5, 5') über seine gesamte Breite eine durchgängige Oberfläche (11, 11') aufweist.

11. Kleidungsstück (2), insbesondere medizinischer Kompressionsstrumpf oder Bandage, aufweisend ein Haftband (1') gemäß einem der vorherigen Ansprüche.

12. Kleidungsstück (2), insbesondere medizinischer Kompressionsstrumpf oder Bandage, nach Anspruch 11, **dadurch gekennzeichnet, dass** das Kleidungsstück (2) durch eine stoffschlüssige Verbindung, oder durch eine formschlüssige Verbindung an dem Haftband (1') befestigt ist.

13. Kleidungsstück (2), insbesondere medizinischer Kompressionsstrumpf oder Bandage, nach Anspruch 11 oder 12, **dadurch gekennzeichnet, dass** nur in dem randseitigen Befestigungsabschnitt (7') des Haftbandes (1') eine Verbindung mit dem Kleidungsstück (2) ausgeführt ist.

14. Kleidungsstück (2), insbesondere medizinischer Kompressionsstrumpf oder Bandage, nach einem der Ansprüche 11 bis 13, **dadurch gekennzeichnet, dass** die Verbindung des Kleidungsstücks (2) mit dem Haftband (1') derart ausgebildet ist, dass die Innenseite des Kleidungsstücks (13) bündig zu dem Haftabschnitt (6') des flexiblen Grundkörpers (3') des Haftbandes (1') angeordnet ist.

15. Kleidungsstück (2), insbesondere medizinischer Kompressionsstrumpf oder Bandage, nach einem der Ansprüche 11 bis 14, **dadurch gekennzeichnet, dass** die Verbindung des Kleidungsstücks (2) mit dem Haftband (1') derart ausgebildet ist, dass die auf dem flexiblen Grundkörper (3') des Haftbandes (1') aufgebrachte rutschhemmende Beschichtung (10) über die Innenseite (13) des Kleidungsstücks (2) hinaussteht.

## Claims

1. An adhesive band (1, 1') for garments (2), in particular for medical compression stockings or bandages, comprising a flexible main body (3, 3') with an inside (4, 4') facing the garment (2) and an outside (5, 5'), the flexible main body (3, 3') comprising a fastening portion (7, 7') for the garment (2) on the inside (4, 4') next to an adhesive portion (6, 6'), **characterised in that** the fastening portion (7, 7') is formed by an edge-side recess (8, 8') in the flexible main body (3, 3'), so that the garment (2) is fastenable to the adhesive band (1, 1'), offset relative to the adhesive portion (6, 6') of the flexible main body (3, 3'), in the recess (8, 8').

2. The adhesive band (1, 1') for garments (2) according to claim 1, **characterised in that** the flexible main body (3, 3') is formed from a textile woven fabric consisting of a number of warp threads and at least one weft thread (9) inserted between the threads.

3. The adhesive band (1) for garments (2) according to claim 2, **characterised in that** the weft thread (9) of the textile woven fabric extends across the entire width of the adhesive band (1, 1').

4. The adhesive band (1, 1') for garments (2) according to any one of the preceding claims, **characterised in that** the flexible main body (3, 3') is elastic in a longitudinal direction and substantially inelastic in a transverse direction.

5. The adhesive band (1) for garments (2) according to any one of the preceding claims, **characterised in that** an anti-slip coating (10) is applied, at least in portions, to the adhesive portion (6) of the flexible main body.

6. The adhesive band (1) for garments (2) according to claim 5, **characterised in that** the anti-slip coating (10) is formed by patterned, in particular knob-shaped or line-shaped adhesive regions.

7. The adhesive band (1, 1') for garments (2) according to any one of the preceding claims, **characterised in that** the width of the recess (8, 8') is at least 5 mm.

8. The adhesive band (1, 1') for garments (2) according to any one of the preceding claims, **characterised in that** the height of the recess (8, 8') is at least 0.5 mm.

9. The adhesive band (1, 1') for garments (2) according to any one of the preceding claims, **characterised in that** the flexible main body (3, 3') is formed in one piece.

10. The adhesive band (1, 1') for garments (2) according to any one of the preceding claims, **characterised in that** the flexible main body (3, 3') on its outside (5, 5') comprises a continuous surface (11, 11') across its entire width.

11. A garment (2), in particular a medical compression stocking or bandage, comprising an adhesive band (1') according to any one of the preceding claims.

12. The garment (2), in particular the medical compression stocking or bandage, according to claim 11, **characterised in that** the garment (2) is fastened to the adhesive band (1') by means of a frictionally engaged connection, or by means of an interlocking connection.

13. The garment (2), in particular the medical compression stocking or bandage, according to claim 11 or 12, **characterised in that** the connection to the garment (2) is made only in the edge-side fastening portion (7') of the adhesive band (1').

14. The garment (2), in particular the medical compression stocking or bandage, according to any one of claims 11 to 13, **characterised in that** the connection between the garment (2) and the adhesive band (1') is designed such that the inside of the garment (13) is arranged flush with the adhesive portion (6') of the flexible main body (3') of the adhesive band (1').

15. The garment (2), in particular the medical compression stocking or bandage, according to any one of claims 11 to 14, **characterised in that** the connection between the garment (2) and the adhesive band (1') is designed such that the anti-slip coating (10) applied to the flexible main body (3') of the adhesive band (1') extends beyond the inside (13) of the garment (2).

## Revendications

1. Ruban adhésif (1, 1') destiné à des vêtements (2), notamment à des bas de compression médicaux ou à des bandages, comportant un corps de base (3, 3') flexible pourvu d'une face interne (4, 4') dirigée vers le vêtement (2) et d'une face externe (5, 5'), le corps de base (3, 3') flexible comportant sur la face interne (4, 4'), à côté d'un segment adhésif (6, 6') un segment de fixation (7, 7') pour le vêtement (2), **caractérisé en ce que** le segment de fixation (7, 7') est conçu par un évidement (8, 8') marginal dans le corps de base (3, 3') flexible, de telle sorte que le vêtement (2) soit susceptible d'être fixé dans l'évidement (8, 8') sur le ruban adhésif (1, 1') avec un décalage par rapport au segment adhésif (6, 6') du corps de base (3, 3') flexible.

2. Ruban adhésif (1, 1') destiné à des vêtements (2) selon la revendication 1, **caractérisé en ce que** le corps de base (3, 3') flexible est formé en un textile tissé, composé de plusieurs fils de chaine et d'au moins un fil de trame (9) incorporé entre les fils.

3. Ruban adhésif (1) destiné à des vêtements (2) selon la revendication 2, **caractérisé en ce que** le fil de trame (9) du textile tissé s'étend sur toute la largeur du ruban adhésif (1, 1').

4. Ruban adhésif (1, 1') destiné à des vêtements (2) selon l'une quelconque des revendications précédentes, **caractérisé en ce que** le corps de base (3, 3') flexible est élastique dans la direction longitudinale et n'est pas élastique dans la direction transversale.

5. Ruban adhésif (1) destiné à des vêtements (2) selon l'une quelconque des revendications précédentes, **caractérisé en ce que** sur le segment adhésif (6) du corps de base flexible est appliqué au moins par endroits un revêtement (10) antidérapant.

6. Ruban adhésif (1) destiné à des vêtements (2) selon la revendication 5, **caractérisé en ce que** le revêtement (10) antidérapant est formé de zones adhésives en forme de motifs, notamment en forme de picots ou en forme de lignes.

7. Ruban adhésif (1, 1') destiné à des vêtements (2) selon l'une quelconque des revendications précédentes, **caractérisé en ce que** la largeur de l'évidement (8, 8') est d'au moins 5 mm.

8. Ruban adhésif (1, 1') destiné à des vêtements (2) selon l'une quelconque des revendications précédentes, **caractérisé en ce que** la hauteur de l'évidement (8,8') est d'au moins 0,5 mm.

9. Ruban adhésif (1, 1') destiné à des vêtements (2) selon l'une quelconque des revendications précédentes, **caractérisé en ce que** le corps de base (3, 3') flexible est conçu en monobloc.

10. Ruban adhésif (1, 1') destiné à des vêtements (2) selon l'une quelconque des revendications précédentes, **caractérisé en ce que** sur sa face externe (5, 5'), le corps de base (3, 3') flexible comporte sur toute sa largeur une surface (11, 11') continue.

11. Vêtement (2), notamment bas de compression médical ou bandage comportant un ruban adhésif (1') selon l'une quelconque des revendications précédentes.

12. Vêtement (2), notamment bas de compression médical ou bandage selon la revendication 11, **caractérisé en ce que** le vêtement (2) est fixé par un assemblage par matière ou par un assemblage par complémentarité de forme sur le ruban adhésif (1').

13. Vêtement (2), notamment bas de compression médical ou bandage selon la revendication 11 ou 12, **caractérisé en ce qu'**uniquement sur le segment de fixation (7') marginal du ruban adhésif (1') est réalisé un assemblage avec le vêtement (2).

14. Vêtement (2), notamment bas de compression médical ou bandage selon l'une quelconque des revendications 11 à 13, **caractérisé en ce que** l'assemblage du vêtement (2) avec le ruban adhésif (1') est conçu de telle sorte que la face interne du vêtement (13) soit placée à fleur du segment adhésif (6') du corps de base (3') flexible du ruban adhésif (1').

15. Vêtement (2), notamment bas de compression médical ou bandage selon l'une quelconque des revendications 11 à 14, **caractérisé en ce que** l'assemblage du vêtement (2) avec le ruban adhésif (1') est conçu de telle sorte que le revêtement (10) antidérapant appliqué sur le corps de base (3') flexible du ruban adhésif (1') déborde au-delà de la face interne (13) du vêtement (2).
